# EUROPEAN PATENT APPLICATION

(11) **EP 1 862 192 A1**
(43) Date of publication of application: **05.12.2007**
(21) Application number: 06715514.3
(22) Date of filing: 10.03.2006
(51) Int. Cl.: A61M 15/00, A61M 11/02

(54) **INHALATOR**

(30) Priority: 18.03.2005 JP 2005079990
(71) Applicant: Omron Healthcare Co., Ltd., Kyoto 615-0084 (JP)
(72) Inventor: NAKATSUJI, Yoshihiro, anoshita-cho, Ukyo-ku, Kyoto-shi, Kyoto (JP); KURATA, Satoshi, anoshita-cho, Ukyo-ku, Kyoto-shi, Kyoto (JP); TABATA, Makoto, anoshita-cho, Ukyo-ku, Kyoto-shi, Kyoto (JP); HAMAGUCHI, Takehiro, anoshita-cho, Ukyo-ku, Kyoto-shi, Kyoto (JP)
(74) Representative: Wilhelms . Kilian & Partner Patentanwälte
(86) International application number: PCT/JP2006/304694
(87) International publication number: WO 2006/100933

(57) **Abstract**

A mouthpiece (160) has a concave portion (161 A) provided in a wall surface defining a flow path, and the concave portion (161 A) is formed to have a curved surface that is curved only in one direction when seen from the above. The concave portion (161 A) is provided with slit-like communication holes (162), and a valve body (170) is fixed by a fixing member (180) to the mouthpiece (160) at a generally central portion of the concave portion (161 A) in the direction in which the concave portion (161 A) is curved, so as to close the communication holes (162). With this structure, even if the valve body deteriorates over time due to long-term use, or the valve body deforms due to long-term use or cleaning and disinfection, its function as an exhalation valve is less likely to be impaired, and as a result, an inhaler capable of preventing leakage of aerosol can be provided.

## Description

### TECHNICAL FIELD

The present invention relates to an inhaler in which liquid reserved in a reservoir is atomized at an atomizing portion, and generated aerosol is conveyed to an oral cavity or nasal cavity of a user using a mouthpiece or the like.

### BACKGROUND ART

An inhaler is a device mainly used for disinfection or treatment of a bronchial tube, including an atomizing portion for atomizing liquid and a conveying portion such as a mouthpiece for conveying the atomized liquid, allowing intake of aerosol generated at the atomizing portion through a user's mouth or nose, using the conveying portion such as the mouthpiece. Of these devices, the inhaler in which medical liquid is aerosolized and conveyed for treatment is particularly referred to as an inhalation treatment device.

Generally, this type of inhaler has an exhalation valve and an inhalation valve provided on the body of the device, so as to allow more efficient inhalation of the aerosol without putting a burden on the user. The exhalation valve and the inhalation valve are each formed as a check valve that includes a communication hole provided in a wall portion forming a flow path for airflow provided inside the device and a valve body attached to the wall portion so as to close the communication hole. The exhalation valve is for discharging exhaled breath of the user from the flow path to the outside of the device, and the inhalation valve is for taking external air from the outside of the device into the flow path to allow the user to inhale air containing the aerosol dispersed in the flow path.

A structure of the exhalation valve described above is known, for example, from the disclosure of Japanese Patent Laying-Open No. 07-185003 or from the disclosure of Japanese Patent Laying-Open No. 11-276587. Further, a structure of the inhalation valve described above is known, for example, from the disclosure of Japanese Patent Laying-Open No. 11-137688.

The exhalation valve disclosed in Japanese Patent Laying-Open No. 07-185003 is formed by attaching a valve body formed of an elastic member to a flat outer wall portion of a mouthpiece. The exhalation valve disclosed in Japanese Patent Laying-Open No. 11-276587 is formed by attaching a valve body formed of a flat-shaped elastic member to a flat-shaped outer wall surface of an orifice portion provided in a mouthpiece. The inhalation valve disclosed in Japanese Patent Laying-Open No. 11-137688 is formed by attaching a valve body formed of an elastic member to a flat inner wall surface inside a device.
Patent Document 1: Japanese Patent Laying-Open No. 07-185003
Patent Document 2: Japanese Patent Laying-Open No. 11-276587
Patent Document 3: Japanese Patent Laying-Open No. 11-137688

### DISCLOSURE OF THE INVENTION

### PROBLEMS TO BE SOLVED BY THE INVENTION

In each of the above structures of attaching the valve bodies, however, a flat-shaped valve body is attached to a flat surface. Accordingly, when the valve body deteriorates over time due to long-term use or deforms due to long-term use, there may be a case where its function of fully closing a communication hole cannot be obtained. In particular, in an inhaler, it is necessary from a hygiene viewpoint to disassemble various parts including the valve body after use and perform cleaning and disinfection, and since the valve body is disassembled with hands, the valve body may be warped or stretched. When such deformation of the valve body is caused, the valve body cannot fulfill its function as a check valve satisfactorily when it is used. As a result, the exhalation valves described above may have a problem that the aerosol leaks from a gap around the exhalation valve, or that external air is introduced from the gap around the exhalation valve when a user inhales air, and airflow is generated only between the exhalation valve and a conveying outlet and thus the user cannot inhale the aerosol dispersed in the flow path. Further, the inhalation valve described above may have a problem that the aerosol leaks from a gap around the inhalation valve, or that external air is introduced from the gap around the inhalation valve when used by a user who inhales a relatively large amount of air, and the amount of inhalation is not limited satisfactorily and thus the user may inhale the aerosol excessively.

Therefore, the present invention was made to solve the above-described problems, and its object is to provide an inhaler in which the function of a valve body as an exhalation valve or an inhalation valve is less likely to be impaired even if the valve body deteriorates over time due to long-term use or the valve body deforms due to long-term use or cleaning and disinfection, and as a result, leakage of aerosol can be prevented.

### MEANS FOR SOLVING THE PROBLEMS

An inhaler according to the present invention includes: a flow path through which airflow passes; an atomizing portion provided in the flow path for atomizing liquid reserved in a reservoir; and a conveying portion for conveying airflow containing aerosol generated at the atomizing portion to an outside of a device, wherein a wall surface defining the flow path is provided with a check valve including a communication hole for establishing communication between the flow path and the outside of the device, and a valve body formed of a flexible member capable of closing the communication hole, and the valve body is attached to the wall portion with being biased.

In the inhaler according to the present invention, the check valve may be an exhalation valve formed by attaching the valve body to an outer wall surface of the wall portion from the outside of the device. The check valve may also be an inhalation valve formed by attaching the valve body to an inner wall surface of the wall portion from a side adjacent to the flow path. Here, the exhalation valve refers to a check valve for discharging exhaled breath introduced into the flow path to the outside of the device, and the inhalation valve refers to a check valve for introducing external air from the outside of the device into the flow path.

Preferably, in the inhaler according to the present invention, a main surface of the wall portion provided with the check valve is provided with a concave portion, the communication hole is provided within the concave portion, and the valve body is attached to a surface of the concave portion. In that case, it is preferable that the concave portion has a surface that is curved only in one of a longitudinal direction and a lateral direction when seen from a side on which the valve body is attached. When the concave portion has the surface that is curved only in one of a longitudinal direction and a lateral direction when seen from the side on which the valve body is attached, it is preferable that a main surface of the valve body that comes into contact with the surface of the concave portion is flat when the valve body is not attached to the wall portion, or curved only in a direction parallel to the direction in which the concave portion is curved and has a curvature smaller than that of the surface of the concave portion when the valve body is not attached to the wall portion.

Preferably, in the inhaler according to present invention, the valve body is fixed to the wall portion at a generally central portion of the surface of the concave portion in the direction in which the surface is curved, or fixed to the wall portion at one end portion of the surface of the concave portion in the direction in which the surface is curved. In that case, it is preferable that the valve body is formed such that its thickness is reduced with increased distance from a fixing position where the valve body is fixed to the wall portion, and that the valve body is fixed to the wall portion by inserting a projected portion provided on the wall portion into a slit provided in the valve body.

Further, in the inhaler according to the present invention, it is preferable as described above that the main surface of the wall portion provided with the check valve is provided with the concave portion, the communication hole is provided within the concave portion, and the valve body is attached to the surface of the concave portion, and in that case, the concave portion may have a surface that is bent only in one of a longitudinal direction and a lateral direction when seen from the side on which the valve body is attached. When the concave portion has the surface that is bent only in one of a longitudinal direction and a lateral direction when seen from the side on which the valve body is attached, it is preferable that the main surface of the valve body that comes into contact with the surface of the concave portion is flat when the valve body is not attached to the wall portion, or bent in a direction parallel to the direction in which the concave portion is bent and an internal angle at a bent portion of the valve body is larger than an internal angle at a bent portion provided in the surface of the concave portion when the valve body is not attached to the wall portion.

Preferably, in the inhaler according to present invention, the valve body is fixed to the bent portion provided in the surface of the concave portion. In that case, it is preferable that the valve body is formed such that its thickness is reduced with increased distance from a fixing position where the valve body is fixed to the wall portion, and that the valve body is fixed to the wall portion by inserting a projected portion provided on the wall portion into a slit provided in the valve body.

Preferably, in the inhaler according to the present invention, a communication hole forming surface defining the communication hole provided in the wall portion has a portion facing the conveying portion, and the portion is provided in an inclined manner such that a distance from the conveying portion is increased as the portion extends from a side adjacent to the flow path to a side adjacent to the outside of the device.

### EFFECTS OF THE INVENTION

According to the present invention, since the valve body is attached to the wall surface of the wall portion forming the flow path with being biased, its function as an exhalation valve or an inhalation valve is less likely to be impaired even if the valve body deteriorates over time due to long-term use or the valve body deforms due to long-term use or cleaning and disinfection, and as a result, leakage of the aerosol can be prevented.

### BRIEF DESCRIPTION OF THE DRAWINGS

Fig. 1 is an external view showing a device structure of an inhaler in accordance with a first embodiment of the present invention.
Fig. 2 is an exploded perspective view showing an assembly structure of a nebulizer of the inhaler shown in Fig. 1.
Fig. 3 is a schematic cross sectional view of the nebulizer shown in Fig. 2 after being assembled.
Fig. 4 is a side view showing a shape of a mouthpiece of the inhaler in accordance with the first embodiment of the present invention.
Fig. 5 is an exploded perspective view of the mouthpiece shown in Fig. 4.
Fig. 6 is a schematic cross sectional view of the mouthpiece taken along the line VI-VI shown in Fig. 4.
Fig. 7 is a schematic cross sectional view of the mouthpiece taken along the line VII-VII shown in Fig. 4.
Fig. 8A schematically shows an assembly structure of a valve body in accordance with the first embodiment of the present invention, illustrating a state before the valve body is assembled.
Fig. 8B schematically shows the assembly structure of the valve body in accordance with the first embodiment of the present invention, illustrating a state after the valve body is assembled.
Fig. 9A schematically shows another example of the assembly structure of the valve body in accordance with the first embodiment of the present invention, illustrating a state before the valve body is assembled.
Fig. 9B schematically shows the other example of the assembly structure of the valve body in accordance with the first embodiment of the present invention, illustrating a state after the valve body is assembled.
Fig. 10 schematically shows still another example of the assembly structure of the valve body in accordance with the first embodiment of the present invention.
Fig. 11 is a schematic cross sectional view of the mouthpiece of the inhaler in accordance with the first embodiment of the present invention.
Fig. 12 is a schematic cross sectional view showing a modification of the mouthpiece of the inhaler in accordance with the first embodiment of the present invention.
Fig. 13 is an exploded perspective view showing a shape of a mouthpiece of an inhaler in accordance with a second embodiment of the present invention.
Fig. 14 is a schematic cross sectional view of the mouthpiece shown in Fig. 13 taken at a portion provided with a crosspiece.
Fig. 15 is a schematic cross sectional view of the mouthpiece shown in Fig. 13 taken at a portion provided with a slit-like communication hole.
Fig. 16A schematically shows an assembly structure of a valve body in accordance with the second embodiment of the present invention, illustrating a state before the valve body is assembled.
Fig. 16B schematically shows the assembly structure of the valve body in accordance with the second embodiment of the present invention, illustrating a state after the valve body is assembled.
Fig. 17A schematically shows another example of the assembly structure of the valve body in accordance with the second embodiment of the present invention, illustrating a state before the valve body is assembled.
Fig. 17B schematically shows the other example of the assembly structure of the valve body in accordance with the second embodiment of the present invention, illustrating a state after the valve body is assembled.
Fig. 18 schematically shows still another example of the assembly structure of the valve body in accordance with the second embodiment of the present invention.
Fig. 19 is a schematic cross sectional view of a nebulizer of an inhaler in accordance with a third embodiment of the present invention.

### DESCRIPTION OF THE REFERENCE SIGNS

1 inhaler, 10 compressor, 20 tube, 100A, 100B nebulizer, 101 flow path, 110 case body, 112 connecting portion, 114 compressed air introducing tube, 116 reservoir, 120 atomizing portion forming body, 122 baffle, 124 liquid suction tube forming portion, 130 flow path forming body, 140 valve body, 142 attachment portion, 150 cap body, 151 concave portion, 152 communication hole, 160 mouthpiece, 160a flow path, 161A, 161B concave portion, 162 communication hole, 163a side surface, 163 crosspiece, 164 projected portion, 168 engagement hole, 170 valve body, 172 slit, 174 retaining member, 180 fixing member, 182 communication hole, 183 crosspiece, 184 retaining portion, 188 engagement projection, 200 liquid.

### BEST MODES FOR CARRYING OUT THE INVENTION

In the following, embodiments of the present invention will be described in detail with reference to the drawings. In the embodiments described below, a compressor type inhaler will be described as an exemplary inhaler.

### First Embodiment

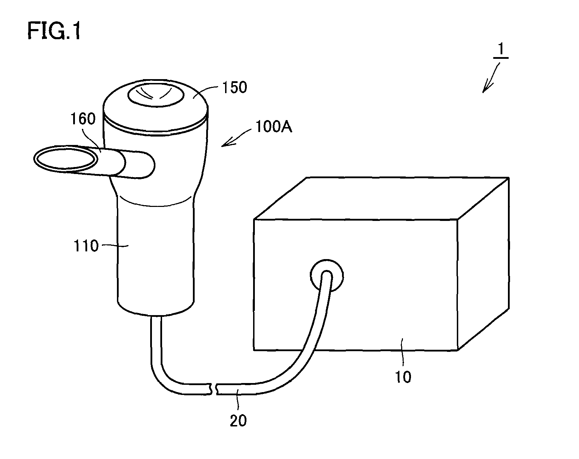

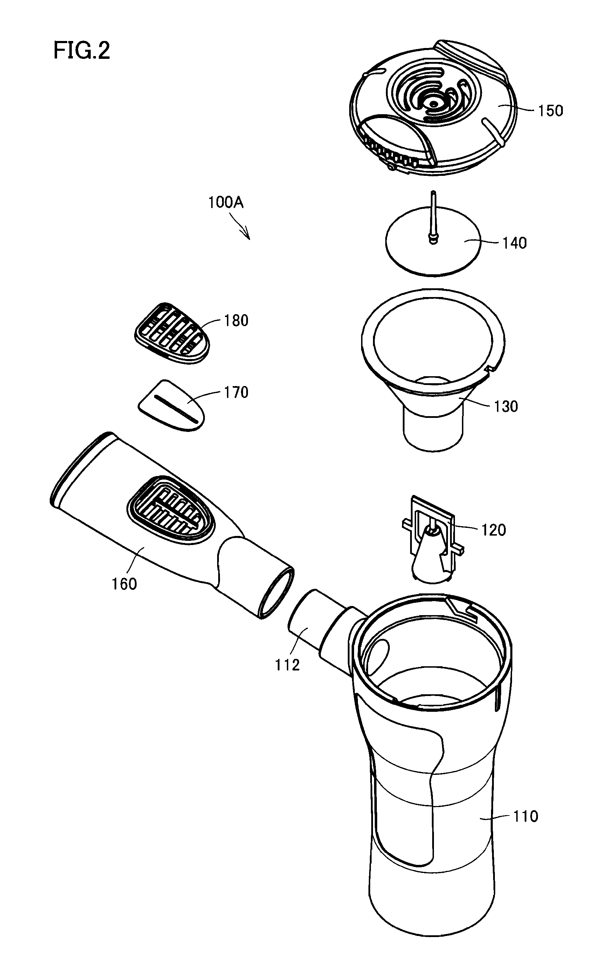

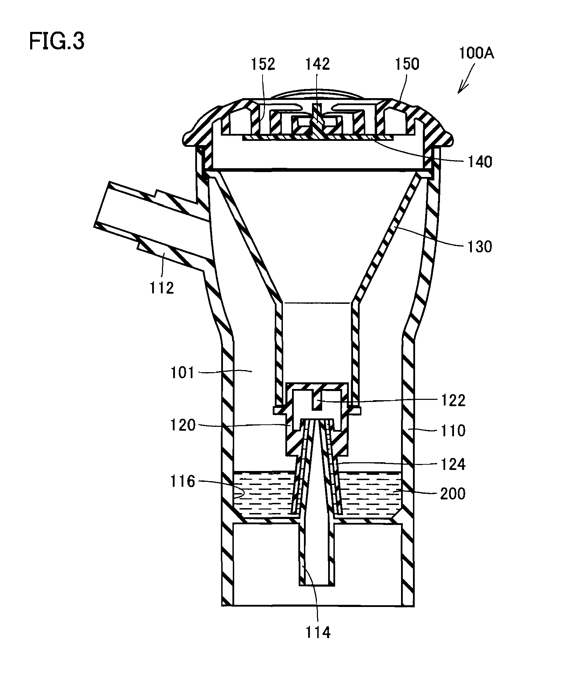

Fig. 1 is an external view showing a device structure of an inhaler in accordance with a first embodiment of the present invention. Fig. 2 is an exploded perspective view showing an assembly structure of a nebulizer of the inhaler shown in Fig. 1. Fig. 3 is a schematic cross sectional view of the nebulizer shown in Fig. 2 after being assembled. In Fig. 3, the mouthpiece shown in Fig. 2 is not shown. Firstly, referring to these drawings, a structure of the inhaler and a structure of the nebulizer, which is a portion of the inhaler, in accordance with the present embodiment will be described.

As shown in Fig. 1, an inhaler 1 in accordance with the present embodiment includes a compressor 10, a tube 20, and a nebulizer 100A. Compressor 10 feeds pressurized compressed air to nebulizer 100A through tube 20.

As shown in Figs. 2 and 3, nebulizer 100A includes a case body 110, an atomizing portion forming body 120, a flow path forming body 130, a valve body 140, and a cap body 150. Case body 110 has a generally cylindrical shape, and atomizing portion forming body 120 and flow path forming body 130 are contained and arranged in case body 110. Valve body 140 is attached to a lower surface of cap body 150, and cap body 150 is attached to case body 110 to close an opening provided at an upper end of case body 110.

Case body 110 has a connecting portion 112 at a predetermined position on its circumferential surface, and a mouthpiece 160 is connected to connecting portion 112. A valve body 170 and a fixing member 180 for fixing valve body 170 to mouthpiece 160 are attached to mouthpiece 160.

As shown in Fig. 3, on a bottom surface of case body 110, a compressed air introducing tube 114 is arranged to extend in an upward/downward direction. Tube 20 described above is attached to a lower tip end of compressed air introducing tube 114. Thus, the compressed air generated by compressor 10 is introduced into compressed air introducing tube 114 through tube 20. An upper tip end of compressed air introducing tube 114 is formed in a tapered shape, and faces a baffle 122 provided on atomizing portion forming body 120, which will be described later. Further, a reservoir 116 is provided around a portion of case body 110 at which compressed air introducing tube 114 is formed. Reservoir 116 is a portion for temporarily reserving liquid 200 such as water, a saline solution, or a medical solution.

A liquid suction tube forming portion 124 of atomizing portion forming body 120 is arranged to face compressed air introducing tube 114 from above. An inner wall surface of liquid suction tube forming portion 124 is arranged to be positioned at a predetermined distance from an outer wall surface of compressed air introducing tube 114, with its lower end arranged to extend close to the bottom surface of reservoir 116 described above. A space between liquid suction tube forming portion 124 and compressed air introducing tube 114 forms a liquid suction tube, and by capillary action, liquid 200 reserved in reservoir 116 is sucked up close to an atomizing portion, which will be described later.

As described above, baffle 122 of atomizing portion forming body 120 is arranged to face the upper tip end of compressed air introducing tube 114, and this portion forms the atomizing portion. At the atomizing portion, the compressed air introduced into compressed air introducing tube 114 by compressor 10 is ejected from the upper tip end of compressed air introducing tube 114 toward baffle 122. At this time, liquid 200 that has been sucked up close to the atomizing portion by capillary action is blown up by negative pressure generated at the atomizing portion, and nebulized, together with the compressed air, toward baffle 122. Because of this function, liquid 200 impinges on baffle 122 to be fine droplets, and the fine droplets are dispersed in airflow passing through a flow path 101 to be aerosol.

Flow path forming body 130 is arranged to be positioned above atomizing portion forming body 120, and flow path 101 through which the airflow passes is formed inside case body 110 by flow path forming body 130. Flow path 101 is in communication with connecting portion 112 formed on the circumferential surface of case body 110 described above, and through this connecting portion 112, the aerosol generated at the atomizing portion is introduced into mouthpiece 160.

Cap body 150 described above has a communication hole 152 at a predetermined position. Valve body 140 is attached to the lower surface of cap body 150. Valve body 140 is attached to cap body 150 by inserting an attachment portion 142 provided at a generally central portion of valve body 140 into an opening formed in cap body 150. Communication hole 152 is provided to establish communication between flow path 101 described above and the outside of nebulizer 100A, and, except for a specific usage state, closed by valve body 140 described above on a side adjacent to flow path 101. Communication hole 152 provided in cap body 150 and valve body 140 attached to the lower surface of cap body 150 form a check valve as an inhalation valve. The inhalation valve is for taking external air from nebulizer 100A to flow path 101 to allow a user to inhale air containing the aerosol dispersed in the flow path.

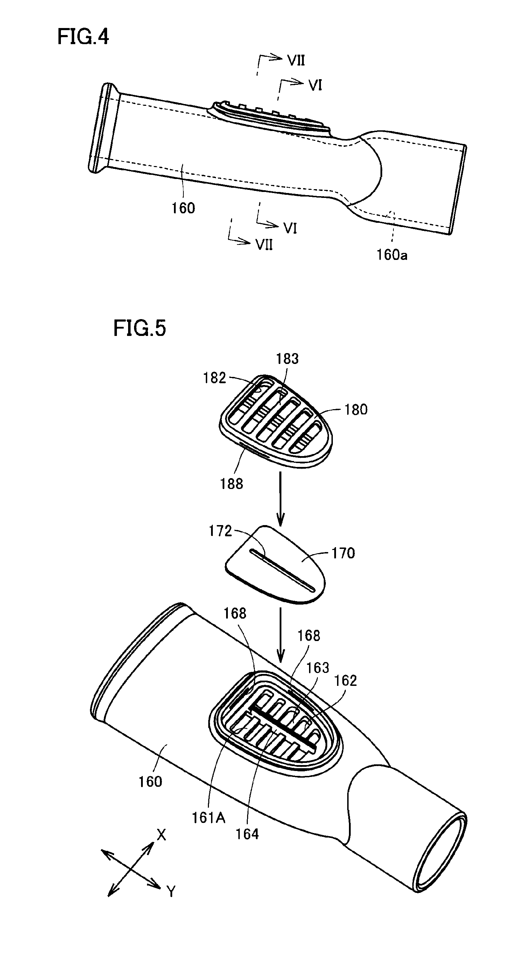

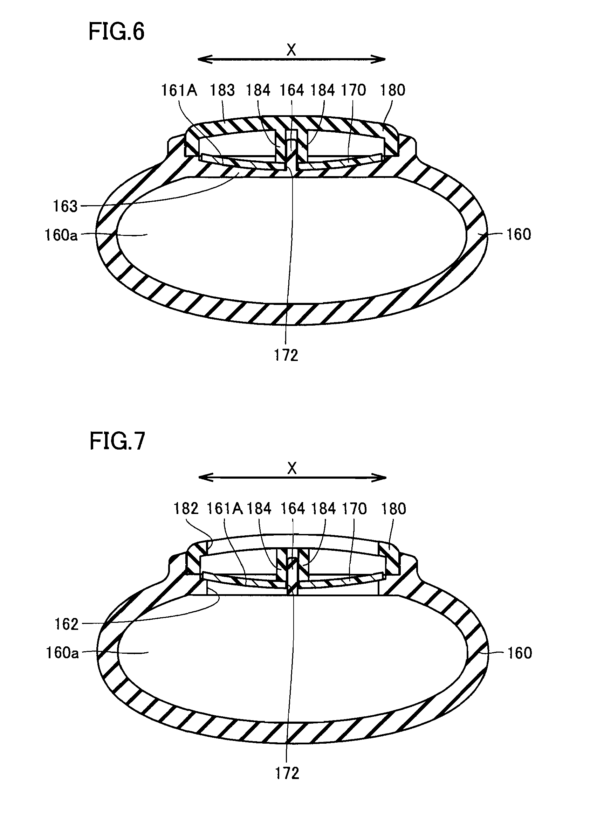

Fig. 4 is a side view showing a shape of the mouthpiece of the inhaler in accordance with the present embodiment, and Fig. 5 is an exploded perspective view of the mouthpiece shown in Fig. 4. Fig. 6 is a schematic cross sectional view of the mouthpiece taken along the line VI-VI shown in Fig. 4, and Fig. 7 is a schematic cross sectional view of the mouthpiece taken along the line VII-VII shown in Fig. 4. Referring to these drawings, a structure of an exhalation valve in accordance with the present embodiment will now be described.

As shown in Figs. 4 and 5, mouthpiece 160 has a shape of a flattened cylinder to fit into the oral cavity of a user. A flow path 160a, which is in communication with flow path 101 provided in case body 110 described above when mouthpiece 160 is connected to case body 110, is provided inside mouthpiece 160. A conveying outlet serving as a conveying portion for conveying the aerosol to the outside of nebulizer 100A is formed at a tip end of flow path 160a. An exhalation valve having a check valve structure is provided on an upper surface of mouthpiece 160. The exhalation valve is for discharging breath exhaled by a user from flow path 160a to the outside of nebulizer 100A.

As shown in Fig. 5, a concave portion 161A is formed on the upper surface of mouthpiece 160. Concave portion 161A has a surface that is curved only in one direction when seen from a side on which valve body 170, which will be described later, is attached. The surface of concave portion 161A shown is curved only in a direction indicated by an arrow X in the drawing, and is not curved in a direction indicated by an arrow Y in the drawing. Specifically, concave portion 161A is formed as a groove having a U-shaped surface in cross section. Further, a communication hole 162 is provided within concave portion 161 A. In mouthpiece 160 shown, a plurality of slit-like communication holes 162 are formed within concave portion 161A, and a crosspiece 163 is formed between adjacent communication holes 162. Slit-like communication hole 162 and crosspiece 163 both extend in the direction indicated by arrow X in the drawing. At a generally central portion of the surface of concave portion 161A in the direction in which the surface is curved (i.e., the direction indicated by arrow X in the drawing), a projected portion 164 is provided to extend in a direction intersecting with the direction in which the surface is curved (i.e., extend in the direction indicated by arrow Y in the drawing).

A valve body 170, formed of highly flexible rubber or resin such as silicon or elastomer, is attached on the surface of concave portion 161 A provided with communication holes 162. Valve body 170 has a slit 172 at its central portion. Slit 172 extends in the direction indicated by arrow Y in the drawing, and is formed slightly larger than an external shape of projected portion 164 described above. Projected portion 164 formed at the generally central portion of the surface of concave portion 161A is inserted into slit 172 provided in valve body 170, and thereby valve body 170 is positioned and attached to mouthpiece 160.

Fixing member 180 for fixing valve body 170 to mouthpiece 160 is attached from above valve body 170. Fixing member 180 is fitted into concave portion 161 A provided in mouthpiece 160 described above, and attached when an engagement projection 188 provided on a side surface thereof is locked by an engagement hole 168 provided in a side surface of concave portion 161 A. In this state, valve body 170 is sandwiched between mouthpiece 160 and fixing member 180. Similar to mouthpiece 160, a plurality of slit-like communication holes 182 are formed in fixing member 180, and a crosspiece 183 is formed between adjacent communication holes 182. Slit-like communication hole 182 and crosspiece 183 both extend in the direction indicated by arrow X in the drawing.

As shown in Fig. 6, in the cross section at a portion provided with crosspiece 163, valve body 170 is pressed onto the surface of concave portion 161A by a retaining portion 184 provided on a lower surface of fixing member 180. Specifically, valve body 170 is pressed and fixed onto mouthpiece 160 on the periphery of slit 172, and, in a portion other than the fixed portion on the periphery of slit 172, valve body 170 is attached to the curved surface of concave portion 161 A with being biased by its own elastic force. Further, as shown in Fig. 7, in the cross section at a portion provided with communication hole 162, valve body 170 is in close contact with the periphery of communication hole 162 by the biasing force described above, and closes communication hole 162.

With the above structure, when the user holds mouthpiece 160 in his or her mouth and inhales air to inhale the aerosol, valve body 170 comes into close contact with the surface of concave portion 161 A, because a pressure outside nebulizer 100A (atmospheric pressure in general) becomes higher than a pressure inside flow path 160a and the biasing force caused by the elastic force of valve body 170 is applied. Thereby, valve body 170 closes communication hole 162 and prevents leakage of the aerosol from communication hole 162. In contrast, when the user exhales breath while holding mouthpiece 160 in his or her mouth, valve body 170 is pushed upward by the pressure inside flow path 160a applied against the biasing force of valve body 170, and communication hole 162 is opened to establish communication between flow path 160a and the outside of nebulizer 100A. Thereby, the exhaled breath inside flow path 160a is discharged to the outside of nebulizer 100A, and thus concentration of the aerosol inside flow path 160a can be kept high, and the user can exhale breath smoothly.

Accordingly, by adopting a structure as described above, valve body 170 is attached with being biased toward a wall surface of a wall portion of mouthpiece 160 forming flow path 160a. Therefore, even if valve body 170 deteriorates over time due to long-term use, or the valve body deforms due to long-term use or cleaning and disinfection, its function as an exhalation valve is less likely to be impaired, and as a result, leakage of the aerosol can be prevented. Further, since valve body 170 can be attached to mouthpiece 160 with a simple structure, disassembly and assembly for cleaning and disinfection can easily be performed.

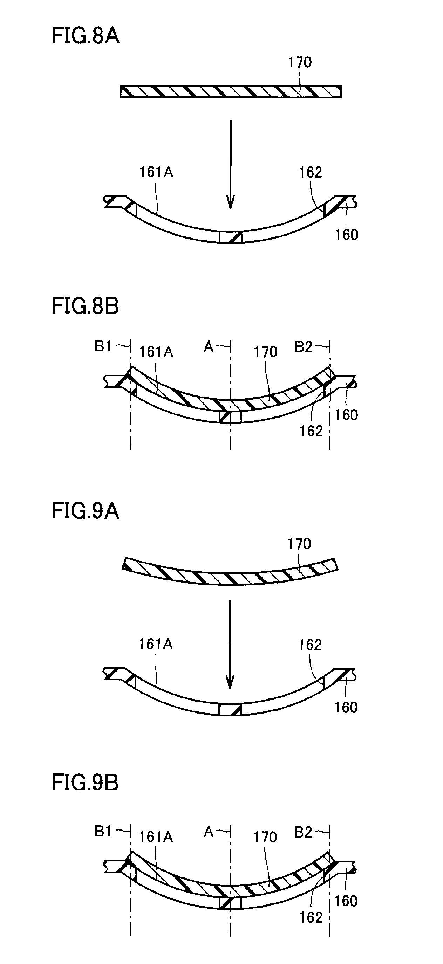

Figs. 8A and 8B schematically show an assembly structure of the valve body described above, in which Fig. 8A illustrates a state before the valve body is assembled, and Fig. 8B illustrates a state after the valve body is assembled. As shown in Fig. 8A, valve body 170 used for inhaler 1 in accordance with the present embodiment is formed to have a flat shape before it is assembled. On the other hand, the surface of concave portion 161A to which valve body 170 is attached is curved only in one direction when seen from the side on which valve body 170 is attached. When valve body 170 is attached to concave portion 161 A having the curved surface, valve body 170 fits onto the curved surface of concave portion 161 A as shown in Fig. 8B, and maintains a tightly closed state reliably. Therefore, there is no need to form valve body 170 to have a three-dimensional shape, and thus a high-performance exhalation valve can be obtained with less manufacturing cost. It is to be noted that valve body 170 and concave portion 161A can be fixed with the biasing state described above kept in good condition, at a position such as the generally central portion of the surface of concave portion 161A in the direction in which the surface is curved (i.e., the portion indicated by a chain line A in Fig. 8B), or one end portion of the surface of concave portion 161 A in the direction in which the surface is curved (i.e., the portion indicated by a chain line B1 or B2 in Fig. 8B).

Figs. 9A and 9B schematically show another example of the assembly structure of the valve body, in which Fig. 9A illustrates a state before the valve body is assembled, and Fig. 9B illustrates a state after the valve body is assembled. Even when valve body 170 is formed three-dimensionally to have a curved shape as shown in Fig. 9A, the close contact of valve body 170 with the surface of concave portion 161 A can be maintained as shown in Fig. 9B if valve body 170 has a curvature smaller than that of the surface of concave portion 161 A. Since the biasing force of valve body 170 applied on concave portion 161A in this case is smaller than that in the assembly structure shown in Figs. 8A and 8B described above, valve body 170 can be opened and closed smoothly even when the amount of breath exhaled by the user is relatively small.

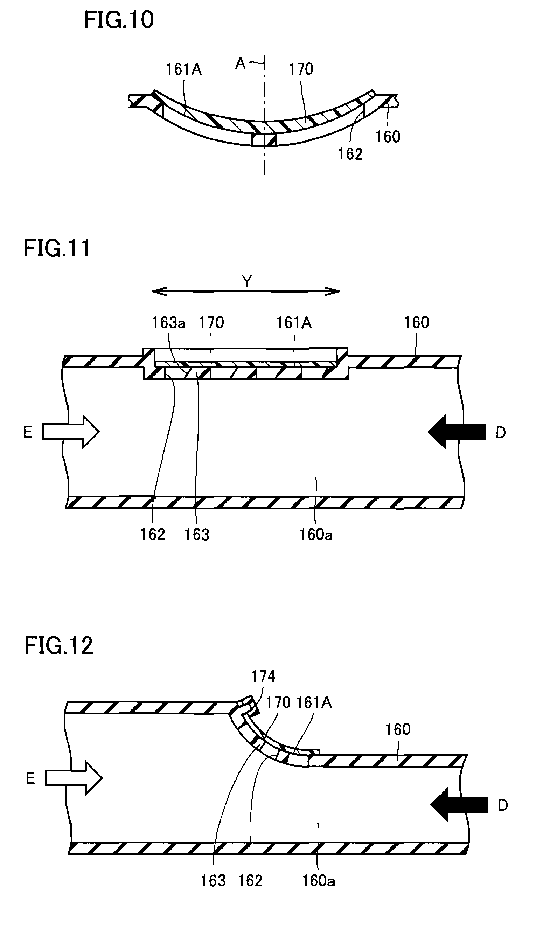

Fig. 10 schematically shows still another example of the assembly structure of the valve body, illustrating a state after the valve body is assembled. In the assembly structure shown in Fig. 10, valve body 170 is formed such that its thickness is reduced with increased distance from the fixing position where valve body 170 is fixed to concave portion 161 A. When valve body 170 has a structure as described above, the biasing force of valve body 170 applied on concave portion 161 A at a position relatively far from the fixing position is smaller than that at a position adjacent to the fixing position, and thus opening and closing of valve body 170 at its end portion can be performed more smoothly. Thereby, valve body 170 can be opened and closed reliably even when the amount of breath exhaled by the user is relatively small.

Fig. 11 is a schematic cross sectional view of the mouthpiece of the inhaler in accordance with the present embodiment, taken in the direction indicated by arrow Y shown in Fig. 5. As shown in Fig. 11, in mouthpiece 160 in accordance with the present embodiment, communication hole 162 is provided in a direction intersecting with a direction in which airflow containing the aerosol passes (a direction indicated by an arrow D in the drawing) and a direction in which the exhaled breath passes (a direction indicated by an arrow E in the drawing). Therefore, side surfaces of crosspiece 163 serve as communication hole forming surfaces defining communication hole 162. Of the side surfaces of crosspiece 163 serving as communication hole forming surfaces, a side surface 163a on a side facing the conveying outlet (i.e., the side surface located downstream in the direction in which the airflow containing the aerosol passes (the direction indicated by arrow D in the drawing)) is formed as an inclined surface. More specifically, side surface 163a is provided in an inclined manner such that a distance from the conveying outlet is increased as side surface 163a extends from a side adjacent to flow path 160a to a side adjacent to the outside of nebulizer 100A. Side surface 163a is formed such that an area of an opening of communication hole 162 on the side adjacent to flow path 160a is smaller than that of an opening of communication hole 162 on the side adjacent to the outside of nebulizer 100A. This structure facilitates flow of the exhaled breath into communication hole 162, and conversely, prevents the airflow containing the aerosol from flowing into communication hole 162, thereby effectively suppressing leakage of the aerosol to the outside of nebulizer 100A through the exhalation valve.

Fig. 12 is a schematic cross sectional view showing a modification of the mouthpiece of the inhaler in accordance with the present embodiment. As shown in Fig. 12, in the present modification, concave portion 161 A provided in mouthpiece 160 has a surface that is curved only in a direction perpendicular to a direction in which the flow path extends, and valve body 170 is attached to the surface of concave portion 161A with being biased. Concave portion 161A is formed to have a step such that flow path 160a has a higher height on a side adjacent to the conveying outlet and has a lower height on a side adjacent to connecting portion 112. Valve body 170 is attached to mouthpiece 160 at one end portion of concave portion 161A on a side adjacent to the conveying outlet in the direction in which concave portion 161A is curved. Specifically, valve body 170 is attached to mouthpiece 160 by inserting a projection provided on a surface of mouthpiece 160 into a hole provided in valve body 170, and fitting a retaining member 174 onto the projection. Since an inner wall surface of concave portion 161A to which valve body 170 is attached is arranged to face the conveying outlet in this structure, this structure also facilitates flow of the exhaled breath into communication hole 162, and conversely, prevents the airflow containing the aerosol from flowing into communication hole 162, thereby effectively suppressing leakage of the aerosol to the outside of nebulizer 100A through the exhalation valve.

### Second Embodiment

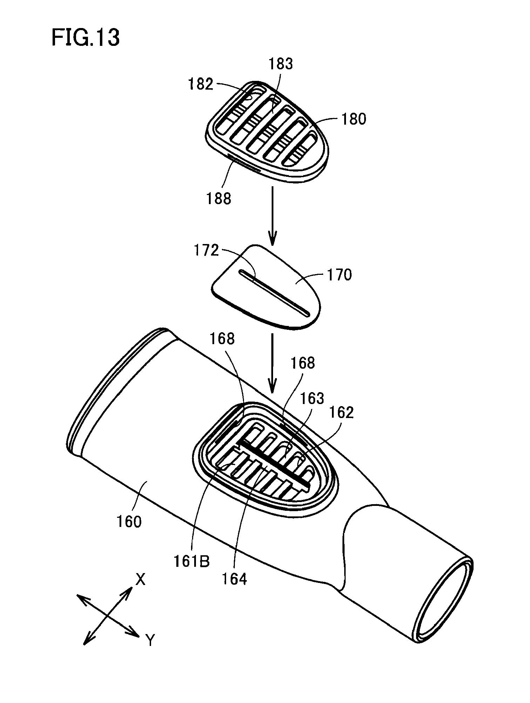

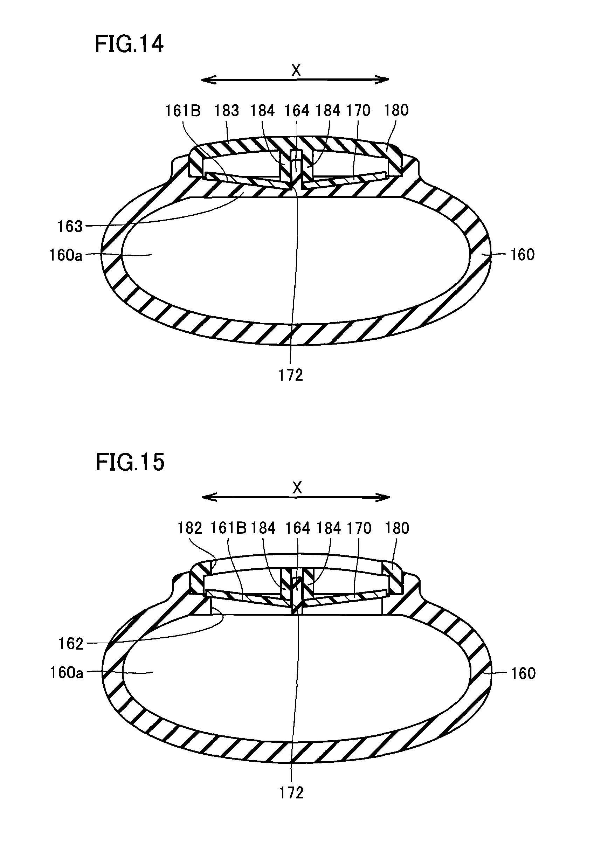

Fig. 13 is an exploded perspective view showing a shape of a mouthpiece of an inhaler in accordance with a second embodiment of the present invention. Fig. 14 is a schematic cross sectional view of the mouthpiece shown in Fig. 13 taken at a portion provided with a crosspiece, and Fig. 15 is a schematic cross sectional view of the mouthpiece shown in Fig. 13 taken at a portion provided with a slit-like communication hole. Parts similar to those in the first embodiment described above are denoted by the same reference characters in the drawings, and description thereof will not be repeated here.

As shown in Fig. 13, mouthpiece 160 of the inhaler in accordance with the present embodiment has a structure almost similar to that in the first embodiment described above, except for the shape of a surface of a concave portion 161B provided on the upper surface of mouthpiece 160. In the mouthpiece in accordance with the first embodiment described above, concave portion 161A is formed to have a U-shaped surface in cross section that is curved only in one direction when seen from the side on which valve body 170 is attached. In contrast, in mouthpiece 160 in accordance with the present embodiment, concave portion 161B is formed to have a V-shaped surface in cross section that is bent only in one direction when seen from the side on which valve body 170 is attached. Specifically, the surface of concave portion 161B is bent in the direction indicated by arrow X in the drawing, and not bent in the direction indicated by arrow Y in the drawing. On a position where the surface is bent, projected portion 164 is provided to extend in a direction intersecting with the direction in which the surface is bent (i.e., in the direction indicated by arrow Y in the drawing).

Also in mouthpiece 160 in accordance with the present embodiment, projected portion 164 formed at a generally central portion of the surface of concave portion 161B is inserted into slit 172 provided in valve body 170, and thereby valve body 170 is positioned and attached to mouthpiece 160, as in the mouthpiece in the first embodiment described above. Further, fixing member 180 for fixing valve body 170 to mouthpiece 160 is attached from above valve body 170.

As shown in Fig. 14, in the cross section at the portion provided with crosspiece 163, valve body 170 is pressed onto the surface of concave portion 161B by retaining portion 184 provided on the lower surface of fixing member 180. Specifically, valve body 170 is pressed and fixed onto mouthpiece 160 on the periphery of slit 172, and, in a portion other than the fixed portion on the periphery of slit 172, valve body 170 is attached to the bent surface of concave portion 161B with being biased by its own elastic force. Further, as shown in Fig. 15, in the cross section at the portion provided with communication hole 162, valve body 170 is in close contact with the periphery of communication hole 162 by the biasing force described above, and closes communication hole 162.

With the above structure, valve body 170 is attached with being biased toward the wall surface of the wall portion of mouthpiece 160 forming flow path 160a, as in the first embodiment described above. Therefore, even if valve body 170 deteriorates over time due to long-term use, or the valve body deforms due to long-term use or cleaning and disinfection, its function as an exhalation valve is less likely to be impaired, and as a result, leakage of the aerosol can be prevented. Further, since valve body 170 can be attached to mouthpiece 160 with a simple structure, disassembly and assembly for cleaning and disinfection can easily be performed.

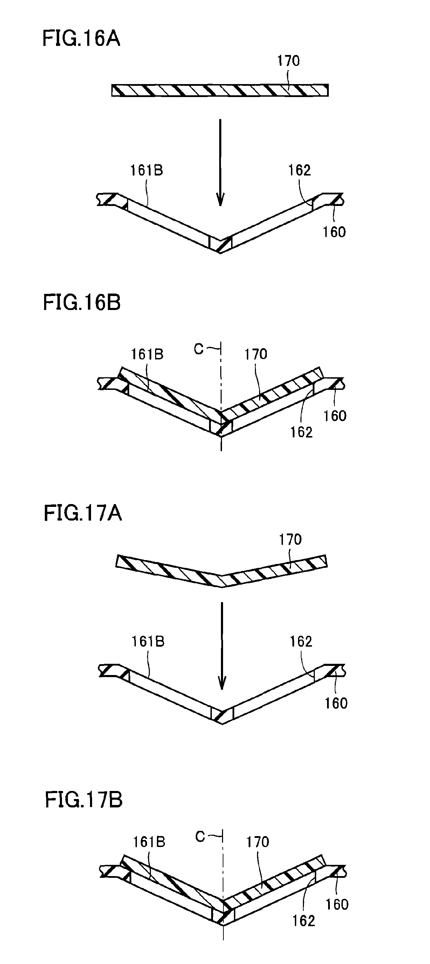

Figs. 16A and 16B schematically show an assembly structure of the valve body described above, in which Fig. 16A illustrates a state before the valve body is assembled, and Fig. 16B illustrates a state after the valve body is assembled. As shown in Fig. 16A, valve body 170 used for inhaler 1 in accordance with the present embodiment is formed to have a flat shape before it is assembled. On the other hand, the surface of concave portion 161B to which valve body 170 is attached is bent only in one direction when seen from the side on which valve body 170 is attached. When valve body 170 is attached to concave portion 161B having the bent surface, valve body 170 fits onto the surface of concave portion 161B as shown in Fig. 16B, and maintains a tightly closed state reliably. Therefore, there is no need to form valve body 170 to have a three-dimensional shape, and thus a high-performance exhalation valve can be obtained with less manufacturing cost. It is to be noted that valve body 170 and concave portion 161B can be fixed with the biasing state described above kept in good condition, at a position such as the generally central portion of the surface of concave portion 161B in the direction in which the surface is bent (i.e., the portion indicated by a chain line C in Fig. 16B), that is, the portion where the surface of concave portion 161B is bent.

Figs. 17A and 17B schematically show another example of the assembly structure of the valve body, in which Fig. 17A illustrates a state before the valve body is assembled, and Fig. 17B illustrates a state after the valve body is assembled. Even when valve body 170 is formed three-dimensionally to have a bent shape as shown in Fig. 17A, the close contact of valve body 170 with the surface of concave portion 161B can be maintained as shown in Fig. 17B if an internal angle at a bent portion of valve body 170 is larger than an internal angle at a bent portion of concave portion 161B. Since the biasing force of valve body 170 applied on concave portion 161B in this case is smaller than that in the assembly structure shown in Figs. 16A and 16B described above, valve body 170 can be opened and closed smoothly even when the amount of breath exhaled by the user is relatively small.

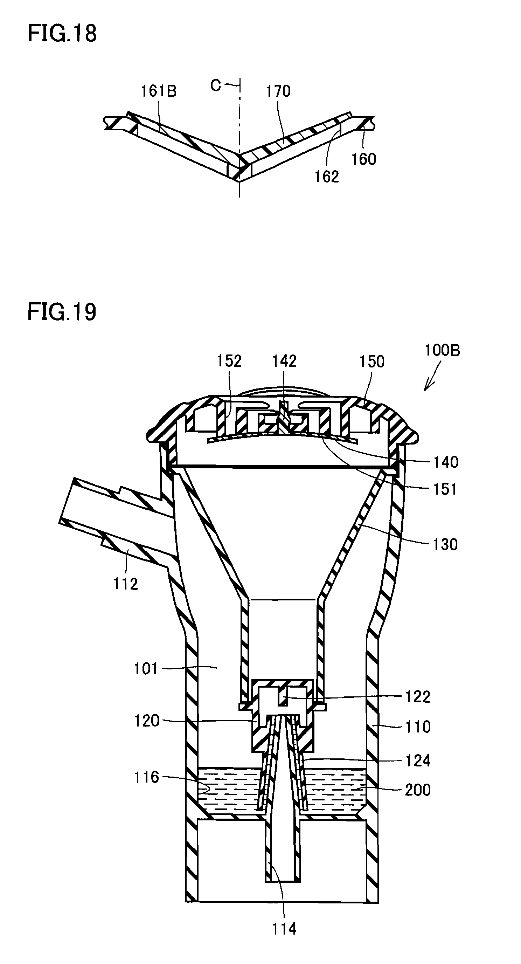

Fig. 18 schematically shows still another example of the assembly structure of the valve body, illustrating a state after the valve body is assembled. In the assembly structure shown in Fig. 18, valve body 170 is formed such that its thickness is reduced with increased distance from the fixing position where valve body 170 is fixed to concave portion 161B. When valve body 170 has a structure as described above, the biasing force of valve body 170 applied on concave portion 161B at a position relatively far from the fixing position is smaller than that at a position adjacent to the fixing position, and thus opening and closing of valve body 170 at its end portion can be performed more smoothly. Thereby, valve body 170 can be opened and closed reliably even when the amount of breath exhaled by the user is relatively small.

### Third Embodiment

Fig. 19 is a schematic cross sectional view of a nebulizer of an inhaler in accordance with a third embodiment of the present invention. In the present embodiment, the assembly structure of the check valve of the present invention is applied to an inhalation valve provided in a nebulizer of an inhaler. Parts similar to those of the nebulizer of the inhaler in accordance with the first embodiment described above are denoted by the same reference characters in the drawings, and description thereof will not be repeated here.

As shown in Fig. 19, in a nebulizer 100B of the inhaler in accordance with the present embodiment, a concave portion 151 is formed in the lower surface of cap body 150. Concave portion 151 has a surface that is curved only in one direction, and valve body 140 is attached to the surface of concave portion 151 with being biased to fit the curved surface. As a structure of assembling valve body 140 to concave portion 151, the assembly structures disclosed in the first and second embodiments described above (the assembly structure shown in Figs. 8A and 8B, the assembly structure shown in Figs. 9A and 9B, the assembly structure shown in Fig. 10, the assembly structure shown in Figs. 16A and 16B, the assembly structure shown in Figs. 17A and 17B, the assembly structure shown in Fig. 18) and the like are applicable.

With the above structure, when the user holds mouthpiece 160 in his or her mouth and inhales air to inhale the aerosol, valve body 140 is pressed downward because a pressure outside nebulizer 100B (atmospheric pressure in general) becomes higher than a pressure inside flow path 101. Thereby, external air can be introduced into flow path 101 efficiently, allowing smooth inhalation of the aerosol. In contrast, when the user exhales breath while holding mouthpiece 160 in his or her mouth, valve body 140 comes into close contact with the surface of concave portion 151 by the biasing force of valve body 140 and the pressure inside flow path 101, and closes communication hole 152 to prevent leakage of the aerosol from communication hole 152.

Accordingly, by adopting a structure as described above, valve body 140 is attached with being biased toward a wall surface of a wall portion of cap body 150 forming flow path 101. Therefore, even if valve body 140 deteriorates over time due to long-term use, or the valve body deforms due to long-term use or cleaning and disinfection, its function as an inhalation valve is less likely to be impaired, and as a result, leakage of the aerosol can be prevented. Further, since valve body 140 can be attached to cap body 150 with a simple structure, disassembly and assembly for cleaning and disinfection can easily be performed.

Although explanation has been given on the case where an exhalation valve to which the present invention is applied is provided to a mouthpiece in the first and second embodiments described above, the exhalation valve does not necessarily have to be provided to a mouthpiece. It may be provided to a case body of a nebulizer, or may also be applied to a mask, a nosepiece, or the like used instead of a mouthpiece. Further, although explanation has been given on the case where an inhalation valve to which the present invention is applied is provided to a cap body attached to an upper portion of a case body of a nebulizer in the third embodiment described above, the inhalation valve does not necessarily have to be provided to the cap body. It may be provided to a case body of a nebulizer, a mouthpiece, a mask, a nosepiece, or the like. To achieve more efficient inhalation of the aerosol, however, the structure as described in each embodiment is most suitable.

Further, although explanation has been given on the case where the present invention is applied to a compressor type inhaler in the above embodiments, the present invention is also applicable to an ultrasonic type inhaler or a so-called ultrasonic vibration type inhaler.

The embodiment as have been described here are mere examples and should not be interpreted as restrictive. The scope of the present invention is determined by each of the claims with appropriate consideration of the written description of the embodiments and embraces modifications within the meaning of, and equivalent to, the languages in the claims.

## Claims

1. An inhaler, comprising:
a flow path through which airflow passes;
an atomizing portion provided in said flow path for atomizing liquid reserved in a reservoir; and
a conveying portion for conveying airflow containing aerosol generated at said atomizing portion to an outside of a device,
wherein a wall portion defining said flow path is provided with a check valve including a communication hole for establishing communication between said flow path and the outside of the device, and a valve body formed of a flexible member capable of closing said communication hole, and
said valve body is attached to said wall portion with being biased.

2. The inhaler according to claim 1, wherein
said check valve is an exhalation valve for discharging exhaled breath introduced into said flow path to the outside of the device, and
said valve body is attached to an outer wall surface of said wall portion from the outside of the device.

3. The inhaler according to claim 1, wherein
said check valve is an inhalation valve for introducing external air from the outside of the device into said flow path, and
said valve body is attached to an inner wall surface of said wall portion from a side adjacent to said flow path.

4. The inhaler according to claim 1, wherein
a main surface of said wall portion provided with said check valve is provided with a concave portion,
said communication hole is provided within said concave portion, and
said valve body is attached to a surface of said concave portion.

5. The inhaler according to claim 4, wherein
said concave portion has a surface that is curved only in one of a longitudinal direction and a lateral direction when seen from a side on which said valve body is attached, and
a main surface of said valve body that comes into contact with the surface of said concave portion is flat when said valve body is not attached to said wall portion.

6. The inhaler according to claim 5, wherein said valve body is fixed to said wall portion at a generally central portion of said surface of said concave portion in the direction in which said surface is curved.

7. The inhaler according to claim 5, wherein said valve body is fixed to said wall portion at one end portion of said surface of said concave portion in the direction in which said surface is curved.

8. The inhaler according to claim 5, wherein said valve body is formed such that its thickness is reduced with increased distance from a fixing position where said valve body is fixed to said wall portion.

9. The inhaler according to claim 5, wherein said valve body is fixed to said wall portion by inserting a projected portion provided on said wall portion into a slit provided in said valve body.

10. The inhaler according to claim 4, wherein
said concave portion has a surface that is curved only in one of a longitudinal direction and a lateral direction when seen from a side on which said valve body is attached, and
a main surface of said valve body that comes into contact with the surface of said concave portion is curved only in a direction parallel to the direction in which said concave portion is curved, and has a curvature smaller than that of the surface of said concave portion, when said valve body is not attached to said wall portion.

11. The inhaler according to claim 4, wherein
said concave portion has a surface that is bent only in one of a longitudinal direction and a lateral direction when seen from a side on which said valve body is attached, and
a main surface of said valve body that comes into contact with the surface of said concave portion is flat when said valve body is not attached to said wall portion.

12. The inhaler according to claim 4, wherein
said concave portion has a surface that is bent only in one of a longitudinal direction and a lateral direction when seen from a side on which said valve body is attached, and
a main surface of said valve body that comes into contact with the surface of said concave portion is bent in a direction parallel to the direction in which said concave portion is bent, and an internal angle at a bent portion of said valve body is larger than an internal angle at a bent portion provided in the surface of said concave portion, when said valve body is not attached to said wall portion.

13. The inhaler according to claim 1, wherein a communication hole forming surface defining said communication hole provided in said wall portion has a portion facing said conveying portion, the portion being provided in an inclined manner such that a distance from said conveying portion is increased as the portion extends from a side adjacent to said flow path to a side adjacent to the outside of the device.
